# EUROPEAN PATENT SPECIFICATION

(11) **EP 3 120 769 B1**
(45) Date of publication and mention of the grant of the patent: **24.10.2018**
(21) Application number: 15766011.9
(22) Date of filing: 27.02.2015
(51) Int. Cl.: A61B 5/1455, A61B 5/021, A61B 5/022

(54) **BIOLOGICAL INFORMATION MEASUREMENT DEVICE AND PULSE OXIMETER**
VORRICHTUNG ZUR MESSUNG BIOLOGISCHER INFORMATIONEN UND PULSOXIMETER
DISPOSITIF DE MESURE D'INFORMATIONS BIOLOGIQUES ET SPHYGMO-OXYMÈTRE

(30) Priority: 19.03.2014 JP 2014055733
(43) Date of publication of application: 25.01.2017
(73) Proprietor: Konica Minolta, Inc., Tokyo 100-7015 (JP)
(72) Inventor: SHIGENAGA, Nobuaki, Tokyo 100-7015 (JP); TATEDA, Norihiro, Tokyo 100-7015 (JP); KAWADA, Kenji, Tokyo 100-7015 (JP); IMAHATA, Kazuo, Tokyo 100-7015 (JP)
(74) Representative: Hoffmann Eitle
(86) International application number: PCT/JP2015/055911
(87) International publication number: WO 2015/141446

(56) References cited:
- EP-A1- 1 217 941
- WO-A1-2010/082444
- WO-A1-2011/052302
- WO-A1-2014/024653
- JP-A- 2007 105 316
- JP-A- 2009 539 576
- US-A1- 2007 287 898

## Description

### Technical Field

The present invention relates to a biological information measurement device and a pulse oximeter.

### Background Art

There is known a pulse oximeter that measures biological information such as oxygen saturation (SpO₂) in blood. According to this pulse oximeter, a measurement part worn on a biological site of a subject radiates light toward the biological site to derive the SpO₂ on the basis of an amount of light transmitted through the biological site or reflected off of the biological site.

Now, a conventional pulse oximeter measuring SpO₂ of a subject over an extended period of time includes one with a configuration in which a probe is worn on a fingertip while a body including a built-in circuit or the like in a casing is worn on an arm. With such configuration, however, a cable connecting the body and the probe tends to get snagged on an obstacle or the like to easily cause problems such as a measurement problem and breakage of the cable.

Accordingly, in order to improve portability without providing the cable connecting the body and the probe, there is proposed a portable pulse oximeter which includes the body and the probe that are integrated and is worn on a finger while holding a fingertip (refer to Patent Literature 1, for example).

Further, Patent Literature 2 and 3 are concerned with an optical device for measurement of blood related signals.

### Citation List

### Patent Literature

Patent Literature 1: U.S. Patent No. 5792052
Patent Literature 2: EP 1 217 941 A, published by WIPO under number WO 01/22868
Patent Literature 3: JP 2007-105316 A.

### Summary of Invention

### Technical Problem

However, the pulse oximeter according to Patent Literature 1 is worn on the fingertip while holding only a distal phalanx of the finger so that the weight concentrates on the distal phalanx, making it difficult for a subject to move the finger as well as making it easy for the pulse oximeter to fall off the finger by inertial force corresponding to the movement of the finger. On the other hand, one can conceive of a configuration that strongly holds the distal phalanx in order to resolve the problem with the pulse oximeter falling off the finger, in which case, however, the blood flow at the distal phalanx can be significantly inhibited after wearing the pulse oximeter for an extended period of time.

Such problem is not limited to the pulse oximeter but is common to a device, such as a photoelectric sphygmograph measuring a pulse, that is worn on the distal phalanx and measures various biological information in general (such device is also referred to as a biological information measurement device).

The preset invention has been made in consideration of the aforementioned problem, where an object of the present invention is to provide a biological information measurement device and a pulse oximeter which ensure sturdiness, less easily fall off the finger, and less easily inhibit the blood flow even after an extended period of measurement.

### Solution to Problem

In order to solve the aforementioned problem, a biological information measurement device according to one aspect is a biological information measurement device as defined in claim 1.

### Brief Description of Drawings

Fig. 1 is a diagram schematically illustrating an exterior of a biological information measurement device according to an embodiment.
Fig. 2 is a diagram schematically illustrating the exterior of the biological information measurement device according to an embodiment.
Fig. 3 is a diagram schematically illustrating an exterior of a body.
Fig. 4 is a diagram schematically illustrating the exterior of the body.
Fig. 5 is a diagram schematically illustrating the exterior of the body.
Fig. 6 is a diagram schematically illustrating the exterior of the body.
Fig. 7 is a diagram schematically illustrating the exterior of the body.
Fig. 8 is a diagram schematically illustrating the exterior of the body.
Fig. 9 is a diagram illustrating a mode in which the body is worn on a finger.
Fig. 10 is a diagram schematically illustrating an exterior of a probe unit.
Fig. 11 is a diagram schematically illustrating the exterior of the probe unit.
Fig. 12 is a diagram schematically illustrating the exterior of the probe unit.
Fig. 13 is a diagram schematically illustrating the exterior of the probe unit.
Fig. 14 is a diagram schematically illustrating the exterior of the probe unit.
Fig. 15 is a diagram schematically illustrating the exterior of the probe unit.
Fig. 16 is a diagram illustrating a mode in which the probe unit is worn on a finger.
Fig. 17 is a block diagram illustrating a functional configuration of the biological information measurement device.
Fig. 18 is a diagram schematically illustrating how the biological information measurement device is worn on a finger.
Fig. 19 is a diagram schematically illustrating how the biological information measurement device is worn on a finger.
Fig. 20 is a diagram schematically illustrating an exterior of a biological information measurement device according to a variation.
Fig. 21 is a diagram schematically illustrating an exterior of a probe unit according to another variation.
Fig. 22 is a diagram schematically illustrating an exterior of a probe unit according to yet another variation.

### Description of Embodiments

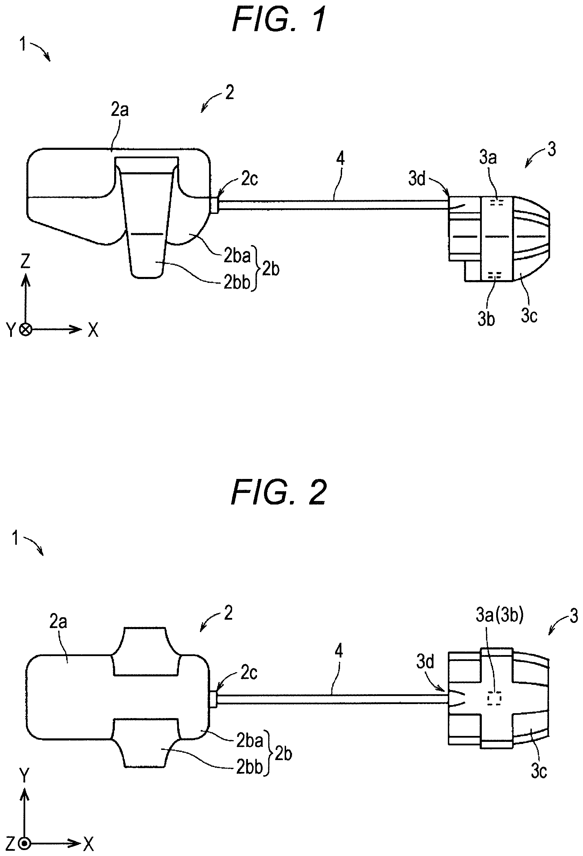

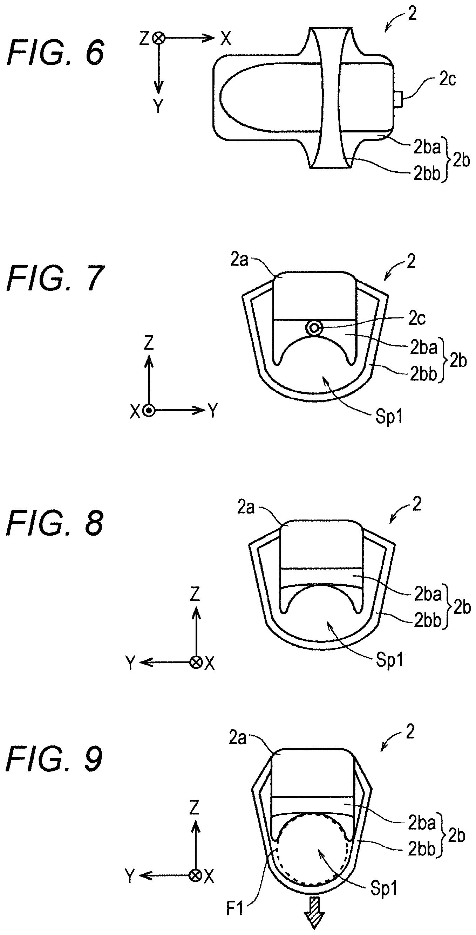

An embodiment as well as various variations according to the present invention will now be described with reference to the drawings. Parts having the same configuration and function among the drawings are assigned the same reference numeral to omit redundant description in the following description. The drawings being schematically illustrated, the size and positional relationship of various structures in each drawing can be modified as appropriate. Each of Figs. 1 to 16 and Figs. 18 to 20 includes a right-handed XYZ coordinate system in which one direction (to the right when Fig. 1 is viewed from the front) of a longitudinal direction of a biological information measurement device 1 corresponds to a +X direction. Moreover, an outer edge of a finger F1 is indicated with a broken line in Figs. 9, 16 and 20.

### <(1) Embodiment>

### <(1-1) Configuration of biological information measurement device>

The biological information measurement device 1 according to an embodiment is a pulse oximeter that acquires information related to at least oxygen saturation in blood within a living body to be examined (also referred to as a subject body) as biological information of the subject body. The subject body in the present embodiment is a human (also referred to as a subject) though it may be an animal other than a human. Then, while a finger of a subject's hand is inserted in space between a light source unit 3a and a light receiving unit 3b, the biological information measurement device 1 acquires the information related to the oxygen saturation by receiving, in the light receiving unit 3b, light emitted from the light source unit 3a and transmitted through the finger.

Figs. 1 and 2 are diagrams each schematically illustrating an exterior of the biological information measurement device 1 according to an embodiment. Fig. 1 is a side view of the biological information measurement device 1, and Fig. 2 is a plan view of the biological information measurement device 1.

As illustrated in Figs. 1 and 2, the biological information measurement device 1 includes a body 2, a probe unit 3 and a connection portion 4, for example. The probe unit 3 is worn on a distal phalanx of a finger of the subject while the body 2 is worn on a part other than the distal phalanx of the finger of the subject, whereby the connection portion 4 connecting the body 2 and the probe unit 3 is reduced in length as well as force applied to hold the distal phalanx is decreased. As a result, the biological information measurement device 1 less easily falls off the finger and less easily inhibits a blood flow even when the biological information measurement device 1 is worn over an extended period of time for measurement.

### <(1-1-1) Body)

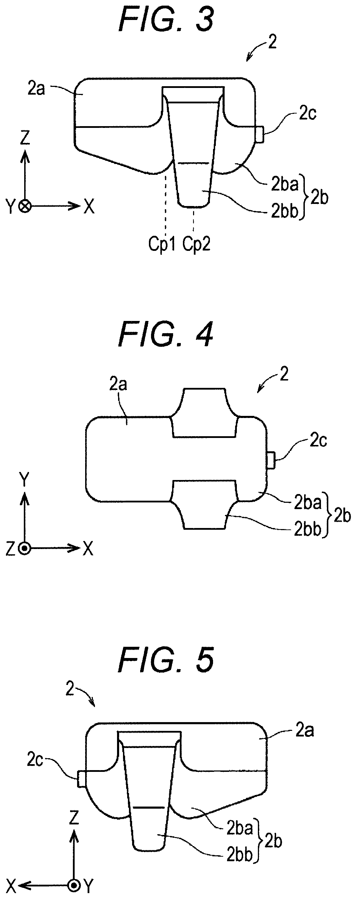

Figs. 3 to 8 are diagrams each schematically illustrating an exterior of the body 2. Fig. 3 illustrates a side of the body 2 facing a -Y direction, Fig. 4 illustrates a side of the body 2 facing a +Z direction, and Fig. 5 illustrates a side of the body 2 facing a +Y direction. Fig. 6 illustrates a side of the body 2 facing a -Z direction, Fig. 7 illustrates a side of the body 2 facing the +X direction, and Fig. 8 illustrates a side of the body 2 facing a -X direction. That is, Figs. 3 to 8 schematically illustrate the exterior of the body 2 when the body 2 is viewed in six directions.

As illustrated in each of Figs. 3 to 8, the body 2 includes a casing 2a, a first fitting portion 2b and a first terminal portion 2c.

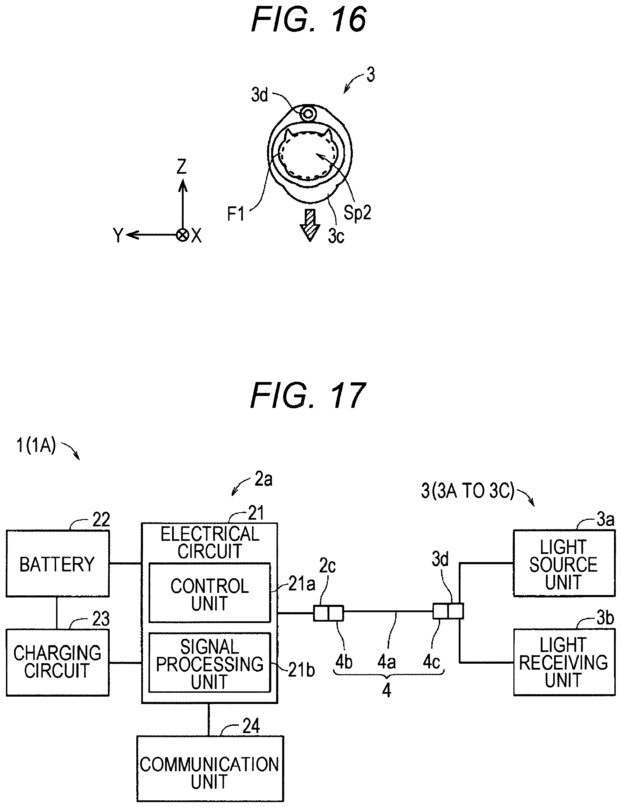

An electrical circuit 21 (Fig. 17) and a battery 22 (Fig. 17) are built in the casing 2a, for example. When the casing 2a is made of material such as plastic that is lightweight and shock-resistant, the electrical circuit 21 and the battery 22 built in the casing 2a are less prone to failure or breakage so that sturdiness of the body 2 can be ensured.

Moreover, the casing 2a is detachably connected to the first fitting portion 2b. The casing 2a not in contact with the finger of the subject can be easily reused as a result. Moreover, the casing 2a can be attached to the first fitting portion 2b only when needed while the first fitting portion 2b is worn on the finger of the subject. The casing 2a can be attached to the first fitting portion 2b by connecting a connection terminal of the casing 2a to a connection terminal provided on a top surface (surface facing the +Z direction) of the first fitting portion 2b, for example.

The first fitting portion 2b attached to the casing 2a is a portion to be worn on a part including at least one of a proximal phalanx and a middle phalanx of the finger of the subject's hand (such part is also referred to as a first fitted portion) . The proximal phalanx corresponds to a part from the base of each of an index finger, a middle finger, a ring finger and a little finger up to its second joint (such part is also referred to as a proximal interphalangeal joint) as well as a part from the base of a thumb up to its first joint. The middle phalanx corresponds to a part from a first joint of each of the index finger, middle finger, ring finger and little finger to its second joint (such part is also referred to as a distal interphalangeal joint). The first fitted portion corresponds to the proximal phalanx in the present embodiment.

Moreover, the first fitting portion 2b is provided to hold the proximal phalanx while the proximal phalanx being the first fitted portion of the finger of the subject's hand extends in the X direction as one direction. Here, the body 2 can be easily worn on the finger when the first fitting portion 2b includes an elastic body exerting elastic force to hold the finger, for example. Polymeric material such as rubber and a spring can be adopted as the elastic body, for example. More specifically, there can be adopted a mode in which substantially the whole first fitting portion 2b is made of resin such as rubber having elasticity, and a mode in which a substantially U-shaped plate spring making up at least a part of the first fitting portion 2b is embedded in resin, for example.

There can be adopted, for example, a mode in which the first fitting portion 2b includes an annular portion (also referred to as a first annular portion) that holds the finger with elastic force. As a result, the force causing the body 2 to be worn on the finger of the subject can be adjusted by changing an inner diameter of the first annular portion and the elastic force according to the size of the subject's finger.

In the present embodiment, as illustrated in Figs. 3 to 8, the first fitting portion 2b includes the first annular portion formed of a finger accommodating portion 2ba and a belt portion 2bb. Here, a first insertion hole Sp1 that is space passing through the first fitting portion 2b in the X direction is formed between the finger accommodating portion 2ba and the belt portion 2bb in the -Z direction of the finger accommodating portion 2ba. The belt portion 2bb includes the elastic body exerting the elastic force to hold the finger.

Fig. 9 is a diagram schematically illustrating a form of the body 2 when the body 2 is worn on the finger F1. Figs. 7 and 8 illustrate a form of the body 2 when the finger F1 is not inserted into the first insertion hole Sp1. Moreover, Fig. 9 illustrates a form of the body 2 when the finger F1 is inserted into the first insertion hole Sp1.

When the body 2 is not worn on the finger F1 as illustrated in Fig. 8, for example, the elastic force exerted by the elastic body of the first fitting portion 2b to hold the finger F1 inserted into the first insertion hole Sp1 causes the first insertion hole Sp1 to undergo elastic deformation toward a direction to be closed in the Z direction.

On the other hand, when the body 2 is worn on the finger F1, the belt portion 2bb undergoes elastic deformation such that the first insertion hole Sp1 is expanded in the -Z direction against the elastic force exerted by the elastic body of the belt portion 2bb. At this time, the finger F1 is held by the first fitting portion 2b when the elastic force exerted by the elastic body of the belt portion 2bb causes the belt portion 2bb to undergo deformation toward the direction to close the first insertion hole Sp1 in the Z direction.

Now, the blood pressure within a capillary of a human (also referred to as a capillary blood pressure) is about 40 gf/cm² where, when a pressure exceeding 40 gf/cm² is externally exerted on a body surface, the human is known to feel pain because of the blood flow in the capillary being inhibited. Therefore, the body 2 can be comfortably worn on the finger F1 by setting the pressing force exerted on the proximal phalanx to be 40 gf/cm² or smaller when the first fitting portion 2b holds the finger F1.

Moreover, the body 2 can be easily worn on the finger F1 in a mode less prone to misalignment when a part of the first fitting portion 2b to be brought into contact with the subject's finger F1 when worn is made of flexible resin. Here, low-hardness rubber in a hardness range of JIS-A20° or lower and sponge-like resin foam can be adopted as the flexible resin, for example. The part to be brought into contact with the finger can be the finger accommodating portion 2ba and the belt portion 2bb, for example. Specifically, there can be adopted a mode in which the belt portion 2bb is made of flexible resin having the thickness of approximately 0.5 mm or thicker and 1.5 mm or thinner, for example.

Now, as illustrated in Fig. 3, a central position (also referred to as a second central position) Cp2 of the first fitting portion 2b in the +X direction is positioned in the +X direction relative to a central position (also referred to as a first central position) Cp1 of the body 2 in the +X direction as one direction. This allows the body 2 to less easily shift from the position at which it is worn on the finger and less easily fall off the finger F1 by the inertial force arising from the movement of the finger F1.

The first terminal portion 2c is a portion to which the connection portion 4 is detachably connected. Accordingly, the size of the first fitting portion 2b as well as the length of the connection portion 4 can be modified easily according to the size of the finger.

### <(1-1-2) Probe unit>

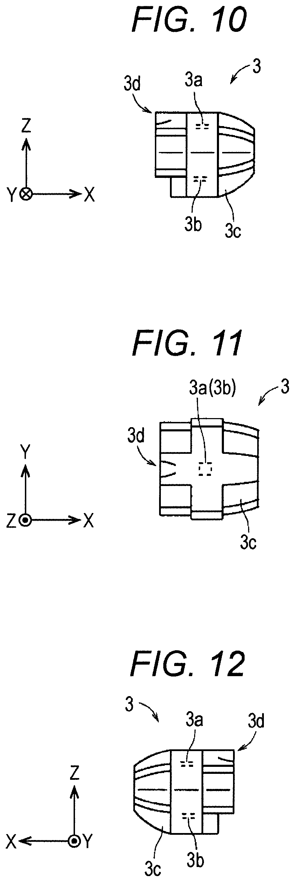

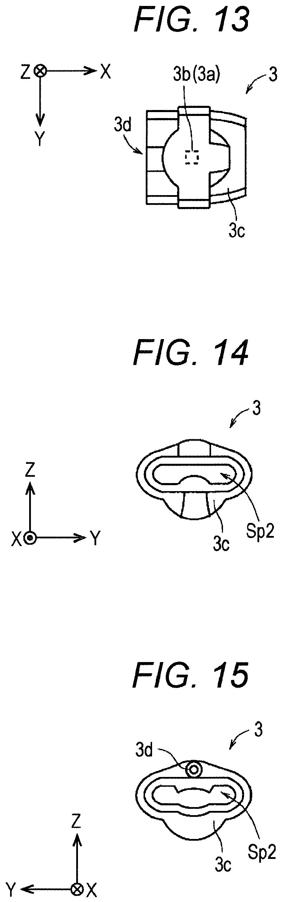

Figs. 10 to 15 are diagrams each schematically illustrating an exterior of the probe unit 3. Fig. 10 illustrates a side of the probe unit 3 facing the -Y direction, Fig. 11 illustrates a side of the probe unit 3 facing the +Z direction, and Fig. 12 illustrates a side of the probe unit 3 facing the +Y direction. Fig. 13 illustrates a side of the probe unit 3 facing the -Z direction, Fig. 14 illustrates a side of the probe unit 3 facing the +X direction, and Fig. 15 illustrates a side of the probe unit 3 facing the -X direction. That is, Figs. 10 to 15 schematically illustrate the exterior of the probe unit 3 when the probe unit 3 is viewed in the six directions.

The probe unit 3 includes a light source unit 3a and a light receiving unit 3b as well as a second fitting portion 3c. The light source unit 3a and the light receiving unit 3b face each other while sandwiching therebetween an area in which the finger F1 is arranged when the second fitting portion 3c is worn on the finger F1. In the present embodiment, the light source unit 3a and the light receiving unit 3b are provided to be exposed on an inner peripheral surface forming a second insertion hole Sp2 of the second fitting portion 3c. On the inner peripheral surface forming the second insertion hole Sp2 of the second fitting portion 3c, a surface facing the -Z direction is provided with the light source unit 3a while a surface facing the +Z direction is provided with the light receiving unit 3b, for example. The probe unit 3 also includes a second terminal portion 3d. The second terminal portion 3d is provided at a portion corresponding to the -X direction of a portion corresponding to the +Z direction of the second fitting portion 3c.

The second fitting portion 3c is to be worn on a portion including the distal phalanx of each finger F1 of the subject's hand (such portion is also referred to as a second fitted portion). The distal phalanx corresponds to a portion from a tip of the finger F1 to its first joint (also referred to as the distal interphalangeal joint). The second fitted portion corresponds to the distal phalanx in the present embodiment.

Moreover, the second fitting portion 3c is provided to hold the distal phalanx while the distal phalanx being the second fitted portion of the finger F1 of the subject's hand extends in the X direction as one direction. Here, the probe unit 3 can be easily worn on the finger F1 when the second fitting portion 3c includes, as the first fitting portion 2b does, an elastic body exerting elastic force to hold the finger F1, for example. Polymeric material such as rubber and a spring can be adopted as the elastic body, for example. More specifically, there can be adopted a mode in which substantially the whole second fitting portion 3c is made of resin such as rubber having elasticity, and a mode in which a substantially U-shaped plate spring making up at least a part of the second fitting portion 3c is embedded in resin, for example.

There can be adopted, for example, a mode in which the second fitting portion 3c includes an annular portion (also referred to as a second annular portion) that holds the finger F1 with elastic force. As a result, the force causing the probe unit 3 to be worn on the finger F1 of the subject can be adjusted by changing an inner diameter of the second annular portion and the elastic force according to the size of the subject's finger F1.

In the present embodiment, as illustrated in Figs. 10 to 15, substantially the whole second fitting portion 3c forms the second annular portion. The second fitting portion 3c includes the second insertion hole Sp2 formed as space passing through the second fitting portion 3c in the X direction.

Fig. 16 is a diagram schematically illustrating a form of the probe unit 3 when the probe unit 3 is worn on the finger F1. Figs. 14 and 15 schematically illustrate a form of the probe unit 3 when the finger F1 is not inserted into the second insertion hole Sp2. Fig. 16 illustrates a form of the probe unit 3 when the finger F1 is inserted into the second insertion hole Sp2.

When the probe unit 3 is not worn on the finger F1 as illustrated in Fig. 15, for example, the elastic force exerted by the elastic body of the second fitting portion 3c to hold the finger F1 inserted into the second insertion hole Sp2 causes the second insertion hole Sp2 to undergo elastic deformation toward a direction to be closed in the Z direction.

On the other hand, when the probe unit 3 is worn on the finger F1, the second fitting portion 3c undergoes elastic deformation such that the second insertion hole Sp2 is expanded in the -Z direction against the elastic force exerted by the elastic body of the second fitting portion 3c, whereby the second fitting portion 3c holds the finger F1 by undergoing deformation toward the direction in which the second insertion hole Sp2 is closed in the Z direction by the elastic force exerted from the elastic body of the second fitting portion 3c.

Here, as described above, a human is known to feel pain when the pressure exceeding 40 gf/cm² is externally applied to the body surface and causes the blood flow in the capillary to be inhibited. Therefore, the pressing force exerted on the distal phalanx is set to 40 gf/cm² or smaller when the second fitting portion 3c holds the finger F1. Assuming a case where the area of a part of the distal phalanx of the finger F1 in contact with the second fitting portion 3c equals 2 cm², for example, the force of 80 gf or less is set to allow the second fitting portion 3c to hold the finger F1. When it is assumed under such condition that the inertial force of up to about 5 G is exerted on the probe unit 3 by active movement of the finger F1, the weight of the probe unit 3 is set to 16 g or lighter which corresponds to one fifth of 80 gf being the maximum value of the force exerted by the second fitting portion 3c to hold the finger F1. The probe unit 3 less easily falls off the finger F1 as a result. Note that the maximum value of inertial force exerted on a load by vibration when a truck transports the load is said to be about 4.5 G.

Moreover, the probe unit 3 can be easily worn on the finger F1 in a manner the finger is less likely to be misaligned when a part of the second fitting portion 3c to be brought into contact with the subject's finger F1 when worn is made of flexible resin. The pressing force exerted by the second fitting portion 3c on the subject's finger F1 can also be adjusted as appropriate. Here, low-hardness rubber in the hardness range of JIS-A20° or lower and sponge-like resin foam can be adopted as the flexible resin, for example. Specifically, there can be adopted a hollow structure formed of flexible resin having the thickness of approximately 0.5 mm or thicker and 1.5 mm or thinner as the second fitting portion 3c, for example.

The second terminal portion 3d is a portion to which the connection portion 4 is detachably connected. Accordingly, the size of the probe unit 3 as well as the length of the connection portion 4 can be modified easily according to the size of the finger. Moreover, a hygienic condition can easily be maintained by adopting a mode in which the probe unit 3 is personalized or a mode in which the probe unit 3 is made disposable. The reuse of the body 2 and the connection portion 4 is also promoted to be able to save resources as well as cut down a medical cost required in using the biological information measurement device 1.

The connection portion 4 electrically connects the body 2 and the probe unit 3. Here, when the connection portion 4 connects the body 2 and the probe unit 3 while allowing a positional relationship between the body 2 and the probe unit 3 to be changed, the subject can move the finger F1 easily to make it easy for the biological information measurement device 1 to be worn on the finger F1 over an extended period of time. There can be adopted, for example, a mode in which the positional relationship between the body 2 and the probe unit 3 is changed by bending of the connection portion 4 in response to the movement of the finger F1 while the biological information measurement device 1 is worn on the subject's finger F1. This allows the subject to bend the finger F1 easily to thus make it easy for the biological information measurement device 1 to be worn on the finger F1 over an extended period of time. The connection portion 4 can be easily bent in response to the movement of the subject's finger F1 when a flexible cable or the like is adopted as the connection portion 4, for example.

Although depending on the length of the subject's finger F1, the connection portion 4 having the length of about 3 cm or longer and 10 cm or shorter allows the finger F1 to be bent while the body 2 is worn on the proximal phalanx of the finger F1 and the probe unit 3 is worn on the distal phalanx of the finger F1. The biological information measurement device 1 can less easily fall off the finger F1 as well as freedom of movement of the finger F1 can be ensured when the connection portion 4 is made as short as possible while allowing the finger F1 to be bent according to the length of the subject's finger F1.

### <(1-2) Functional configuration of biological information measurement device>

Fig. 17 is a block diagram illustrating a functional configuration of the biological information measurement device 1.

As illustrated in Fig. 17, the biological information measurement device 1 includes the light source unit 3a, the light receiving unit 3b, the electrical circuit 21, the battery 22, a charging circuit 23, a communication unit 24, and the connection portion 4. Here, the connection portion 4 includes an extension portion 4a, a third terminal portion 4b and a fourth terminal portion 4c, for example. The extension portion 4a electrically connects the third terminal portion 4b and the fourth terminal portion 4c.

The light source unit 3a is electrically connected to the second terminal portion 3d. The second terminal portion 3d is electrically connected to the fourth terminal portion 4c of the connection portion 4, the third terminal portion 4b of the the connection portion 4 is electrically connected to the first terminal portion 2c, and the first terminal portion 2c is electrically connected to the electrical circuit 21. The light source unit 3a is thus electrically connected to the electrical circuit 21. The light source unit 3a emits light toward the light receiving unit 3b with power supplied from the battery 22 according to control performed by the electrical circuit 21. The light source unit 3a includes a portion emitting light of wavelength λ1 in a red region and a portion emitting light of wavelength λ2 in an infrared region. A Light Emitting Diode (LED) can be adopted as the light source unit 3a, for example. Note that in measurement, red light of the wavelength A1 and infrared light of the wavelength λ2 are alternately emitted from the light source unit 3a.

The light receiving unit 3b is electrically connected to the second terminal portion 3d. The light receiving unit 3b is thus electrically connected to the electrical circuit 21. The light receiving unit 3b outputs a current signal with magnitude corresponding to intensity of the received light to a signal processing unit 21b to be described. Note that the light receiving unit 3b includes a photoelectric conversion element such as a silicon photodiode that is sensitive to at least the red light of the wavelength λ1 and the infrared light of the wavelength λ2, for example. Then while the distal phalanx of the finger F1 is inserted into the second insertion hole Sp2, for example, the light receiving unit 3b receives a portion of the light of the wavelengths λ1 and λ2 emitted from the light source unit 3a and transmitted through a biological tissue of the finger F1.

In measuring biological information, the red light of the wavelength λ1 and the infrared light of the wavelength λ2 are alternately emitted from the light source unit 3a, then the light receiving unit 3b performs a light receiving operation in synchronization with a light emitting operation of the light source unit 3a. The light emitting operation of the light source unit 3a and the light receiving operation of the light receiving unit 3b can be controlled by a control unit 21a to be described. The light projecting/receiving operation pertaining to the red light and the infrared light is repeated at a cycle of about no less than 1/100 (seconds) and no greater than 1/30 (seconds), for example.

Note that when the light source unit 3a and the light receiving unit 3b are implemented on flexible printed circuits (FPC), for example, the light source unit 3a and the light receiving unit 3b can be easily incorporated into the biological information measurement device 1.

The electrical circuit 21 includes the control unit 21a and the signal processing unit 21b. The electrical circuit 21 can be formed of various electronic components, an integrated circuit component and a central processing unit (CPU), for example.

The control unit 21a controls an operation of each of the light source unit 3a and the light receiving unit 3b. Under control of the control unit 21a, for example, the light source unit 3a alternately emits the red light of the wavelength λ1 and the infrared light of the wavelength λ2 each at the cycle of 1/100 (seconds), for example. The control unit 21a also controls data communication of the communication unit 24.

In the signal processing unit 21b, the current signal periodically output from the light receiving unit 3b is converted into a voltage signal, which is then amplified. The voltage signal refers to an analog signal pertaining to pulse wave (such signal is also referred to as a pulse wave signal) . Here, the signal processing unit 21b converts the analog pulse wave signal into a digital pulse wave signal to obtain a digital value of the pulse wave. That is, the digital value of the pulse wave is obtained on the basis of the current signal output from the light receiving unit 3b when the light receiving unit 3b receives the light emitted from the light source unit 3a and transmitted through the finger. The signal processing unit 21b at this time analyzes data on the basis of the digital pulse wave signal. As a result, various values are calculated including the amount of the red light and infrared light received in the light receiving unit 3b, an amplitude of the pulse wave, a ratio of an amplitude of the red light to an amplitude of the infrared light, an oxygen saturation value (SpO₂ value) in blood, a pulse rate and an interval (cycle) of the pulse wave.

Note that the electrical circuit 21 may include various memories storing data that is acquired by the signal processing unit 21b.

The battery 22 includes a secondary battery, for example. A nickel-hydrogen battery or a lithium-ion battery can be adopted as the secondary battery, for example. The battery 22 supplies power to various structures such as the light source unit 3a and the electrical circuit 21 included in the biological information measurement device 1. The body 2 thus does not require a mechanism to exchange a primary battery such as a dry battery. As a result, the body 2 having simple and sturdy structure can be realized. Note that there can also be adopted a configuration in which the battery 22 is the primary battery.

The charging circuit 23 is a circuit provided to charge the secondary battery of the battery 22. There can be adopted a mode in which the secondary battery is charged by connecting a feeding unit not shown to a terminal electrically connected to the secondary battery, for example. This allows the secondary battery to be charged with a simple configuration. In the present embodiment, for example, the connection portion 4 can be detached from the first terminal portion 2c to electrically connect the feeding unit to the first terminal portion 2c. Then while the feeding unit is electrically connected to the first terminal portion 2c, power is supplied from the feeding unit to the battery 22 as the secondary battery through the first terminal portion 2c to charge the battery 22. When the first terminal portion 2c is used to be connected to both the connection portion 4 and the feeding unit as described above, a charging terminal need not be additionally installed to thus be able to simplify the configuration of the biological information measurement device 1. In other words, the biological information measurement device 1 can be reduced in size.

The communication unit 24 transmits the data acquired by the signal processing unit 21b in a wireless or wired manner. Accordingly, there can be adopted a mode in which the biological information measurement device 1 is not provided with a configuration analyzing and saving a signal as well as a display unit displaying a measurement result. As a result, the biological information measurement device 1 can be reduced in size and save power as well as requires less manufacturing cost. Here, for example, there can be adopted a mode in which the communication unit 24 transmits data to an external device through the first terminal portion 2c while the external device is electrically connected to the first terminal portion 2c through a cable or the like. When there is adopted the configuration in which the communication unit 24 transmits the data acquired by the signal processing unit 21b in the wireless manner, the body 2 does not require a configuration to be connected to the external device so that the data can be easily transmitted to the external device without inhibiting the movement of the finger F1 or the like.

Moreover, the communication unit 24 may also be adapted to receive a signal from the external device and output the signal to the control unit 21a. At this time, there may be adopted a mode in which various controls and various computations pertaining to the control unit 21a and the signal processing unit 21b are executed by the control unit 21a in response to the signal from the external device.

Note that there may also be adopted a mode in which an operation unit (not shown) is provided in the body 2 so that the various controls and various computations pertaining to the control unit 21a and the signal processing unit 21b are executed according to a signal that is input in response to an operation of the operation unit. The operation unit can include a power button, a measurement start button, and a measurement end button, for example. The power button is a button provided to switch power to be supplied and not supplied from the battery 22 to each unit of the biological information measurement device 1. The measurement start button is a button provided to start measurement of the oxygen saturation value (SpO₂ value) in blood or the like. The measurement end button is a button provided to end the measurement of the SpO₂ value in blood or the like.

### <(1-3) Biological information measurement device worn on finger>

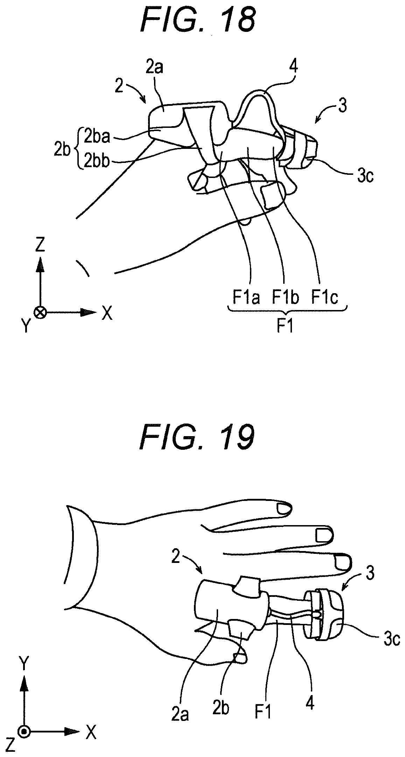

Figs. 18 and 19 are diagrams schematically illustrating how the biological information measurement device 1 is worn on the subject's finger F1. Figs. 18 and 19 illustrate a case in which the biological information measurement device 1 is worn on an index finger as the finger F1 of the subject. The finger F1 includes a proximal phalanx F1a, a middle phalanx F1b, and a distal phalanx F1c, for example.

The tip of the finger F1 is inserted into the first insertion hole Sp1 of the first fitting portion 2b then into the second insertion hole Sp2 of the second fitting portion 3c, for example, so that the body 2 is worn on the proximal phalanx F1a while the probe unit 3 is worn on the distal phalanx F1c as illustrated in Figs. 18 and 19. At this time, for example, the finger F1 is inserted into the insertion hole Sp2 such that light emitted from the light source unit 3a is radiated onto an area between a fingernail and the distal interphalangeal joint (first joint) of the distal phalanx F1c inserted into the second insertion hole Sp2. Moreover, the connection portion 4 is at this time arranged at a position facing a portion of the finger F1 facing outside when the finger F1 is bent (such portion is also referred to as a dorsal portion) . When the joint of the finger F1 is not bent but stretched, for example, the connection portion 4 is bent and at the same time a substantially central part of the connection portion 4 is away from the dorsal portion of the finger F1. When the joint of the finger F1 is bent, for example, the connection portion 4 is bent along the finger F1.

Here, the proximal phalanx F1a is held by the first fitting portion 2b while the proximal phalanx F1a being the first fitted portion extends in the +X direction as one direction. At this time, as illustrated in Figs. 3 and 18, the second central position Cp2 of the first fitting portion 2b in the +X direction as one direction is positioned closer to the distal phalanx F1c than the first central position Cp1 of the body 2 in the +X direction as one direction. Accordingly, the inertial force arising from the movement of the finger F1 is less easily generated against the body 2. This as a result allows the biological information measurement device 1 to less easily shift from the position at which it is worn on the finger and less easily fall off the finger F1 by the inertial force arising from the movement of the finger F1.

Moreover, the biological information measurement device 1 can be easily worn on the finger F1 in the manner the device less easily shifts from the position at which it is worn on the finger, when a part of the first fitting portion 2b and the second fitting portion 3c to be brought into contact with the subject's finger F1 when worn is made of flexible resin.

### <(1-4) Summary of embodiment>

In the biological information measurement device 1 according to the present embodiment described above, the body 2 and the probe unit 3 are electrically connected by the connection portion 4 where the probe unit 3 is worn on the distal phalanx F1c of the subject' s finger F1, while the body 2 is worn on the proximal phalanx F1a of the subject's finger F1. This allows the connection portion 4 connecting the body 2 and the probe unit 3 to be reduced in length and allows the force holding the distal phalanx F1c to be reduced when the biological information measurement device 1 is worn on the distal phalanx F1c. As a result, the biological information measurement device 1 less easily falls off the finger F1 and less easily inhibits the blood flow even when the biological information measurement device 1 is worn over an extended period of time for measurement. Moreover, the electrical circuit 21 and the battery 22 are built into the casing 2a of the body 2 so that sturdiness of the biological information measurement device 1 can be ensured as well. That is, there can be realized the biological information measurement device 1 which ensures the sturdiness, less easily falls off the finger F1, and less easily inhibits the blood flow even when the device is worn over an extended period of time for measurement.

### <(2) Variation>

The present invention is not to be limited to the aforementioned embodiment, where various modifications and improvements can be made without departing from the appended claims.

While the aforementioned embodiment adopts the configuration in which the connection portion 4 is arranged at the position facing the dorsal portion of the finger F1 when the first fitting portion 2b and the second fitting portion 3c are worn on the finger F1, for example, it is not limited to such configuration. The connection portion 4 may also be arranged at a position facing any of a portion positioned inside when the finger F1 is bent (such portion is also referred to as a ventral portion), the dorsal portion, and a lateral portion connecting the ventral portion and the dorsal portion of the finger F1, for example.

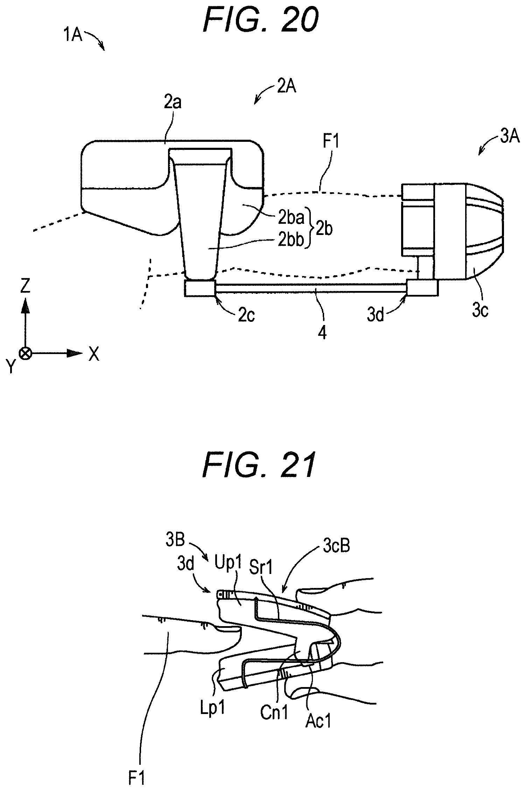

As illustrated in Fig. 20, for example, there can be adopted a configuration in which a connection portion 4 is arranged along a ventral portion of a finger F1 when a first fitting portion 2b and a second fitting portion 3c are worn on the finger F1. There can be adopted in this case a biological information measurement device 1A where the body 2 of the biological information measurement device 1 is replaced by a body 2A in which a first terminal portion 2c is attached to a belt portion 2bb of the first fitting portion 2b, and the probe unit 3 of the biological information measurement device 1 is replaced by a probe unit 3A in which a second terminal portion 3d is arranged at a different position. Such configuration allows the connection portion 4 to be positioned on a palm side when the finger F1 is bent so that the connection portion 4 less easily gets snagged on another structure and that the biological information measurement device 1A less easily falls off the finger F1.

Moreover, while each of the first and second fitting portions 2b and 3c of the aforementioned embodiment includes the annular portion to hold the finger F1 with the elastic force, it is not limited to such configuration. The first and second fitting portions 2b and 3c may also be configured to include at least one of the annular portion, a clip portion holding the finger F1 with elastic force, and a band portion wrapped around the finger F1 to be worn on the finger F1, for example. When such configuration is adopted, the force with which the biological information measurement device 1 is worn on the finger F1 can be adjusted according to the size of the finger F1 as well.

Fig. 21 illustrates a probe unit 3B which is based on the probe unit 3 and in which the second fitting portion 3c including the annular portion is replaced by a second fitting portion 3cB including a clip portion. In the probe unit 3B, a protrusion Cnl provided in a substantially rectangular parallelepiped first casing Up1 is turnably connected to a hinge portion Ac1 of a substantially rectangular parallelepiped second casing Lp1. Moreover, an elastic portion Sr1 such as a spring causes elastic force to be exerted between the first casing Up1 and the second casing Lp1 such that a first one end of the first casing Up1 and a second one end of the second casing Lp1 approach each other. Then, a first another end opposite to the first one end of the first casing Up1 and a second another end opposite to the second one end of the second casing Lp1 are pinched by a finger or the like to bring the first another end and the second another end closer to each other, so that the first casing Up1 can be turned about the hinge portion Ac1. Note that a light source unit 3a is arranged in the first casing Up1 while a light receiving unit 3b is arranged in the second casing Lp1, for example.

According to such second fitting portion 3cB, for example, external force is applied to be able to turn the first casing Up1 about the hinge portion Ac1 and separate the first one end of the first casing Up1 from the second one end of the second casing Lp1 against the elastic force exerted by the elastic portion Sr1. A finger F1 is then inserted into space between the first one end and the second one end and, once the external force is removed, the elastic force by the elastic portion Sr1 causes the first casing Up1 to turn about the hinge portion Ac1 such that the first one end of the first casing Up1 and the second one end of the second casing Lp1 approach each other. The finger F1 can thus be held by the first casing Up1 and the second casing Lp1.

When such configuration is adopted, the force with which the biological information measurement device is worn on the finger F1 of a subject can be adjusted by adjusting the elastic force of the elastic portion Sr1 as appropriate according to the size of the subject's finger F1.

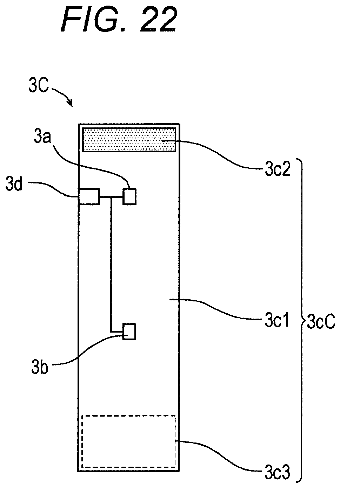

Fig. 22 illustrates a probe unit 3C which is based on the probe unit 3 and in which the second fitting portion 3c including the annular portion is replaced by a second fitting portion 3cC including a band portion. In the probe unit 3C, the second fitting portion 3cC includes a band-like body 3c1, a first fixing member 3c2 and a second fixing member 3c3.

A thin flexible strip made of cloth or resin can be adopted as the band-like body 3c1, for example. A light source unit 3a, a light receiving unit 3b and a second terminal portion 3d are arranged in the band-like body 3c1 where wiring is used to electrically connect the light source unit 3a and the second terminal portion 3d as well as the light receiving unit 3b and the second terminal portion 3d. Here, flexible printed circuits on which the light source unit 3a, the light receiving unit 3b and the second terminal portion 3d are disposed may be adopted on the band-like body 3c1, for example.

Moreover, hook and loop fasteners that can be stuck and unstuck to/from each other can be adopted as the first fixing member 3c2 and the second fixing member 3c3, for example. In this case, for example, there can be adopted a mode in which the first fixing member 3c2 is a portion raised to form a loop while the second fixing member 3c3 is a portion raised to form a hook. The probe unit 3C can thus be worn on a distal phalanx F1c by sticking the first fixing member 3c2 and the second fixing member 3c3 together while the band-like body 3c1 is wrapped around the distal phalanx F1c. Note that instead of the hook and loop fasteners, there may be adopted a pair of adjusters used to adjust the length and having a mechanism similar to a waist belt worn at an upper end of pants.

When such configuration is adopted, the force with which the biological information measurement device is worn on the finger F1 of a subject can be adjusted by properly adjusting the force applied to fasten the distal phalanx with the band-like body 3c1 according to the size of the subject's finger F1.

Note that the annular portion of each of the first fitting portion 2b and the second fitting portion 3c may be replaced by a substantially annular portion that is not completely annular and partly discontinuous.

While the casing 2a is detachably connected to the first fitting portion 2b in the aforementioned embodiment, it is not limited to such configuration. That is, the casing 2a may be formed integrally with the first fitting portion 2b, for example.

Moreover, while the biological information measurement device 1 is worn on the finger F1 of the subject's hand in the aforementioned embodiment, it is not limited to such configuration. That is, the biological information measurement device 1 may be worn on a toe of the subject, for example.

Moreover, while the body 2 and the probe unit 3 in the aforementioned embodiment are worn on the proximal phalanx and the distal phalanx of one finger F1, respectively, it is not limited to such configuration. That is, the body 2 and the probe unit 3 may be worn on different fingers, for example. There can be adopted a mode in which, for example, the body 2 is worn on a proximal phalanx of a first finger of one hand of the subject while the probe unit 3 is worn on a distal phalanx of a second finger of the hand. The first finger and the second finger may be two adjacent fingers or two non-adjacent fingers, for example. Moreover, there may be adopted a mode in which the body 2 is worn on proximal phalanxes of two or more adjacent fingers while the probe unit 3 is worn on distal phalanxes of two or more adjacent fingers, for example. That is, the body 2 can be worn on the proximal phalanx of one or more fingers of a subject body while the probe unit 3 can be worn on the distal phalanx of one or more fingers of the subject body, for example.

While the communication unit 24 is provided in the aforementioned embodiment, it is not limited to such configuration. There may be adopted, for example, a mode in which a storage is provided instead of the communication unit 24 so that data stored in the storage is read by the external device connected to the first terminal portion 2c.

Moreover, while the portion of the first and second fitting portions 2b and 3c that is brought into contact with the finger F1 when worn on the subject's finger is made of flexible resin in the aforementioned embodiment, it is not limited to such configuration. That is, for example, the portion of the first and second fitting portions 2b and 3c that is brought into contact with the finger F1 when worn on the subject's finger may be formed of a flexible bag-like member into which a filler such as gas, liquid or gel is injected.

Moreover, while the connection portion 4 is flexible and bendable in the aforementioned embodiment, it is not limited to such configuration. That is, for example, the connection portion 4 may be formed of a plurality of rod-like members that is connected to one another and be bent at a portion at which the rod-like members are connected to one another. Moreover, for example, the connection portion 4 may be adapted to connect the body 2 and the probe unit 3 such that the positional relationship between the body 2 and the probe unit 3 can be changed by adopting a mechanism in which a protrusion slides in a slot and a turnable hinge portion. The positional relationship between the body 2 and the probe unit 3 can be changed by adopting any of the configurations, whereby the subject can move the finger F1 easily to make it easy for the biological information measurement device 1 to be worn on the finger F1 over an extended period of time. The connection portion 4 may also be highly rigid and unbendable, for example. However, the connection portion 4 made bendable allows the subject to bend the finger F1 easily to thus make it easy for the biological information measurement device 1 to be worn on the finger F1 over an extended period of time.

Moreover, while the first fitting portion 2b is worn on the proximal phalanx F1a in the aforementioned embodiment, it is not limited to such configuration. The first fitting portion 2b may also be worn on the middle phalanx F1b, for example . That is, the first fitting portion 2b may be worn on a portion including at least one of the proximal phalanx F1a and the middle phalanx F1b of the finger F1. Accordingly, the body 2 may be worn on a first fitted portion including at least one of the proximal phalanx and the middle phalanx of one or more fingers of the subject body while the probe unit 3 may be worn on a second fitted portion including the distal phalanx of one or more fingers of the subject body. Note that when the first fitting portion 2b is worn on the proximal phalanx F1a, the inertial force generated in the body 2 by moving a hand is reduced so that the body 2 is less easily shifted from the position by which it is worn and less easily falls off the finger F1. The first fitting portion 2b may also be provided to reach the top of a joint connecting the proximal phalanx F1a and a metacarpal bone or the vicinity of the joint, for example. There can be adopted a mode in which the finger accommodating portion 2ba is provided to reach the top of the joint connecting the proximal phalanx F1a and the metacarpal bone or the vicinity of the joint, for example. This increases an area of contact between the first fitting portion 2b and the subject's hand so that the biological information measurement device 1 can be more stably worn on the finger F1.

Moreover, while the signal processing unit 21b of the biological information measurement device 1 acquires the digital value of the oxygen saturation in blood (SpO₂ value) in the aforementioned embodiment, it is not limited to such configuration. That is, for example, there may be adopted a biological information measurement device other than the pulse oximeter that does not acquire the SpO₂ value but measures biological information related to the pulse wave or the like such as a heart rate.

Note that all or a part of the configurations making up each of the aforementioned embodiment and various variations can be combined as appropriate to the extent the consistency is maintained.

### Reference Signs List

1, 1A Biological information measurement device
2, 2A Body
2a Casing
2b First fitting portion
2ba Finger accommodating portion
2bb Belt portion
2c First terminal portion
3, 3A, 3B, 3C Probe unit
3a Light source unit
3b Light receiving unit
3c, 3cB, 3cC Second fitting portion
3d Second terminal portion
4 Connection portion
4a Extension portion
4b Third terminal portion
4c Fourth terminal portion
21 Electrical circuit
21a Control unit
21b Signal processing unit
22 Battery
23 Charging circuit
24 Communication unit
Cp1 First central position
Cp2 Second central position
F1 Finger
F1a Proximal phalanx
F1b Middle phalanx
F1c Distal phalanx

## Claims

1. A biological information measurement device (1) configured to acquire biological information of a subject body by receiving, in a light receiving unit (3b), light emitted from a light source unit (3a) while a finger (F1) of the subject body is inserted into space between the light source unit (3a) and the light receiving unit (3b), the device comprising:
a body (2); a probe unit (3); and a connection portion (4) which electrically connects the body (2) and the probe unit (3), wherein
the body (2) includes: a casing (2a) in which a battery (22) and an electrical circuit (21) are incorporated; and a first fitting portion (2b) which is attached to the casing (2a) and is configured to be worn on a first fitted portion including a portion of at least one of a proximal phalanx (F1a) and a middle phalanx (F1b) of one or more fingers of the subject body,
the probe unit (3) includes:
the light source unit (3a) and the light receiving unit (3b); and a second fitting portion (3c) which is configured to be worn on a second fitted portion including a distal phalanx (F1c) of one or more fingers of the subject body, and
the body (2) further includes a first terminal portion (2c) to which the connection portion (4) is detachably connected,
**characterized in that**
the battery (22) includes a secondary battery, and
in a state in which the connection portion (4) is detached from the first terminal portion (2c) to electrically connect a feeding unit to the first terminal portion (2c), power can be supplied from the feeding unit to the secondary battery through the first terminal portion (2c) to charge the secondary battery.

2. The biological information measurement device (1) according to claim 1, wherein
the first fitting portion (2b) is provided to hold the first fitted portion while the first fitted portion extends in one direction, and
a first central position (Cp1) of the body (2) in the one direction is positioned farther from the distal phalanx (F1c) than a second central position (Cp2) of the first fitting portion (2b) in the one direction when the first fitting portion (2b) is worn on the first fitted portion.

3. The biological information measurement device (1) according to claim 1 or 2, wherein
the first fitting portion (2b) and the second fitting portion (3c) include at least one of an annular portion which is configured to hold one or more fingers of the subject body with elastic force, a band portion which is configured to be wrapped around one or more fingers of the subject body and to be worn on one or more fingers of the subject body, and a clip portion which is configured to hold one or more fingers of the subject body with elastic force.

4. The biological information measurement device (1) according to claim 3, wherein
a portion of the first fitting portion (2b) and the second fitting portion (3c) that is brought into contact with one or more fingers of the subject body when worn on the one or more fingers of the subject body is made of flexible resin.

5. The biological information measurement device (1) according to any one of claims 1 to 4, wherein
the probe unit (3) includes a second terminal portion (3d) to which the connection portion (4) is detachably connected.

6. The biological information measurement device (1) according to any one of claims 1 to 5, wherein
the casing (2a) is detachably connected to the first fitting portion (2b).

7. The biological information measurement device (1) according to any one of claims 1 to 6, wherein
the connection portion (4) connects the body (2) and the probe unit (3) in a manner that a positional relationship between the body (2) and the probe unit (3) can be changed.

8. The biological information measurement device (1) according to claim 7, wherein
the connection portion (4) is configured to be bent to change the positional relationship between the body (2) and the probe unit (3).

9. The biological information measurement device (1) according to claim 8, wherein
when the first fitting portion (2b) and the second fitting portion (3c) are worn on one or more fingers of the subject body, the connection portion (4) is configured to be arranged along a ventral portion of the finger (F1) that is positioned inside when the finger (F1) is bent.

10. The biological information measurement device (1) according to any one of claims 1 to 9, wherein
the body (2) further includes a communication unit (24) configured to transmit data in a wireless manner.

11. A pulse oximeter configured as the biological information measurement device (1) according to any one of claims 1 to 10 and
configured to acquire information on at least oxygen saturation in blood of the subject body as the biological information.

## Patentansprüche

1. Vorrichtung (1) zur Messung biologischer Informationen, ausgelegt zum Erfassen biologischer Informationen eines Körpers eines Subjekts durch Empfangen in einer Lichtempfangseinheit (3b) von einer Lichtquelleneinheit (3a) emittiertem Licht, während ein Finger (F1) des Körpers eines Subjekts in den Raum zwischen der Lichtquelleneinheit (3a) und der Lichtempfangseinheit (3b) eingeführt ist, wobei die Vorrichtung umfasst:
einen Körper (2); eine Sondeneinheit (3); und einen Verbindungsabschnitt (4), der den Körper (2) und die Sondeneinheit (3) elektrisch verbindet,
wobei
der Körper (2) beinhaltet: ein Gehäuse (2a), in das eine Batterie (22) und eine elektrische Schaltung (21) integriert sind; und einen ersten Befestigungsabschnitt (2b), der an dem Gehäuse (2a) angebracht ist und ausgelegt ist, an einem ersten Befestigungsabschnitt getragen werden, der einen Abschnitt von mindestens einem proximalen Fingerglied (F1a) und einem mittleren Fingerglied (F1b) von einem oder mehreren Fingern des Körpers eines Subjekts enthält,
wobei die Sondeneinheit (3) beinhaltet:
die Lichtquelleneinheit (3a) und die Lichtempfangseinheit (3b); und einen zweiten Befestigungsabschnitt (3c), der ausgelegt ist, an einem zweiten Befestigungsabschnitt getragen zu werden, der ein distales Fingerglied (F1c) von einem oder mehreren Fingern des Körpers eines Subjekts beinhaltet, und
der Körper (2) weiter einen ersten Endabschnitt (2c) beinhaltet, mit dem der Verbindungsabschnitt (4) lösbar verbunden ist,
**dadurch gekennzeichnet, dass**
die Batterie (22) einen Akkumulator beinhaltet, und
in einem Zustand, in dem der Verbindungsabschnitt (4) von dem ersten Endabschnitt (2c) getrennt ist, um eine Zuführeinheit mit dem ersten Endabschnitt (2c) elektrisch zu verbinden, Strom von der Zuführeinheit zu dem Akkumulator durch den ersten Endabschnitt (2c) zugeführt werden kann, um den Akkumulator zu laden.

2. Vorrichtung (1) zur Messung biologischer Informationen nach Anspruch 1, wobei der erste Befestigungsabschnitt (2b) vorgesehen ist, um den ersten Befestigungsabschnitt zu halten, während sich der erste Befestigungsabschnitt in eine Richtung erstreckt, und
eine erste zentrale Position (Cp1) des Körpers (2) in die eine Richtung weiter von dem distalen Fingerglied (F1c) als eine zweite zentrale Position (Cp2) von dem ersten Befestigungsabschnitt (2b) in die eine Richtung positioniert wird, wenn der erste Befestigungsabschnitt (2b) an dem ersten befestigten Abschnitt getragen wird.

3. Vorrichtung (1) zur Messung biologischer Informationen nach Anspruch 1 oder 2, wobei
der erste Befestigungsabschnitt (2b) und der zweite Befestigungsabschnitt (3c) mindestens eines enthalten von einem ringförmigen Abschnitt , der ausgelegt ist zum Halten eines oder mehrerer Finger des Körpers eines Subjekts mit elastischer Kraft, einem Bandabschnitt, der ausgelegt ist, um um einen oder mehrere Finger des Körpers eines Subjekts gewickelt zu werden und an einem oder mehreren Fingern des Körpers eines Subjekts getragen zu werden, und einem Clipabschnitt, der ausgelegt ist, einen oder mehrere Finger des Körpers eines Subjekts mit elastischer Kraft zu halten.

4. Vorrichtung (1) zur Messung biologischer Informationen nach Anspruch 3, wobei ein Abschnitt des ersten Befestigungsabschnitts (2b) und des zweiten Befestigungsabschnitts (3c), der mit einem oder mehreren Fingern des Körpers eines Subjekts in Kontakt gebracht wird, wenn er an dem einen oder den mehreren Fingern des Körpers eines Subjekts getragen wird, aus biegsamem Harz besteht.

5. Vorrichtung (1) zur Messung biologischer Informationen nach einem der Ansprüche 1 bis 4, wobei die Sondeneinheit (3) einen zweiten Endabschnitt (3d) beinhaltet, mit dem der Verbindungsabschnitt (4) lösbar verbunden ist.

6. Vorrichtung (1) zur Messung biologischer Informationen nach einem der Ansprüche 1 bis 5, wobei das Gehäuse (2a) mit dem ersten Befestigungsabschnitt (2b) lösbar verbunden ist.

7. Vorrichtung (1) zur Messung biologischer Informationen nach einem der Ansprüche 1 bis 6, wobei der Verbindungsabschnitt (4) den Körper (2) und die Sondeneinheit (3) so verbindet, dass eine Positionsbeziehung zwischen dem Körper (2) und der Sondeneinheit (3) geändert werden kann.

8. Vorrichtung (1) zur Messung biologischer Informationen nach Anspruch 7, wobei der Verbindungsabschnitt (4) dazu ausgestattet ist, gebogen zu werden, um die Positionsbeziehung zwischen dem Körper (2) und der Sondeneinheit (3) zu ändern.

9. Vorrichtung (1) zur Messung biologischer Informationen nach Anspruch 8, wobei wenn der erste Befestigungsabschnitt (2b) und der zweite Befestigungsabschnitt (3c) an einem oder mehreren Fingern des Körpers eines Subjekts getragen werden, der Verbindungsabschnitt (4) ausgelegt ist, um entlang eines ventralen Abschnitts des Fingers (F1) angeordnet zu sein, der sich im Inneren befindet, wenn der Finger (F1) gebogen wird.

10. Vorrichtung (1) zur Messung biologischer Informationen nach einem der Ansprüche 1 bis 9, wobei der Körper (2) weiter eine Kommunikationseinheit (24) beinhaltet, die ausgelegt ist, um Daten in einer drahtlosen Weise zu übertragen.

11. Pulsoximeter, ausgelegt als die Vorrichtung (1) zur Messung biologischer Informationen nach einem der Ansprüche 1 bis 10 und ausgelegt zum Erfassen von Informationen hinsichtlich mindestens der Sauerstoffsättigung im Blut des Körpers eines Subjekts als die biologischen Informationen.

## Revendications

1. Dispositif de mesure d'informations biologiques (1) configuré pour acquérir des informations biologiques d'un sujet en recevant, dans une unité de réception de lumière (3b), une lumière émise par une unité de source de lumière (3a) tandis qu'un doigt (F1) du corps du sujet est inséré dans un espace entre l'unité de source de lumière (3a) et l'unité de réception de lumière (3b), le dispositif comprenant :
un corps (2); une unité de sonde (3) ; et une partie de connexion (4) qui relie électriquement le corps (2) et l'unité de sonde (3),
dans lequel
le corps (2) inclut : un boîtier (2a) dans lequel une batterie (22) et un circuit électrique (21) sont incorporés ; et une première partie d'ajustement (2b) qui est attachée au boîtier (2a) et est configurée pour être portée sur une première partie ajustée incluant une partie d'au moins une parmi une phalange proximale (F1a) et une phalange centrale (F1b) d'un ou plusieurs doigts du corps du sujet,
l'unité de sonde (3) inclut :
l'unité de source de lumière (3a) et l'unité de réception de lumière (3b) ; et une seconde partie d'ajustement (3c) qui est configurée pour être portée sur une seconde partie ajustée incluant une phalange distale (F1c) d'un ou plusieurs doigts du corps du sujet, et
le corps (2) inclut en outre une première partie terminale (2c) à laquelle la partie de connexion (4) est connectée de manière détachable,
**caractérisé en ce que**
la batterie (22) inclut une batterie secondaire, et dans un état dans lequel la partie de connexion (4) est détachée de la première partie terminale (2c) pour connecter électriquement une unité d'alimentation à la première partie terminale (2c), le courant peut-être fourni de l'unité d'alimentation à la batterie secondaire à travers la première partie terminale (2c) pour charger la batterie secondaire.

2. Dispositif de mesure d'informations biologiques (1) selon la revendication 1, dans lequel
la première partie d'ajustement (2b) est prévue pour maintenir la première partie ajustée tandis que la première partie ajustée s'étend dans une direction, et
une première position centrale (Cp1) du corps (2) dans la première direction est positionnée plus loin de la phalange distale (F1c) qu'une seconde position centrale (Cp2) de la première partie d'ajustement (2b) dans la première direction lorsque la première partie d'ajustement (2b) est portée sur la première partie ajustée.

3. Dispositif de mesure d'informations biologiques (1) selon la revendication 1 ou 2, dans lequel
la première partie d'ajustement (2b) et la deuxième partie d'ajustement (3c) incluent au moins une partie annulaire qui est configurée pour maintenir un ou plusieurs doigts du corps du sujet avec une force élastique, une partie de bande qui est configurée pour être enroulée autour d'un ou plusieurs doigts du corps du sujet et pour être portée sur un ou plusieurs doigts du corps du sujet, et une partie de clip qui est configurée pour maintenir un ou plusieurs doigts du corps du sujet avec une force élastique.

4. Dispositif de mesure d'informations biologiques (1) selon la revendication 3, dans lequel
une partie de la première partie d'ajustement (2b) et de la seconde partie d'ajustement (3c) qui est amenée en contact avec un ou plusieurs doigts du corps du sujet lorsqu'il est porté sur un ou plusieurs doigts du corps du sujet est fabriquée en résine flexible.

5. Dispositif de mesure d'informations biologiques (1) selon l'une quelconque des revendications 1 à 4, dans lequel
l'unité de sonde (3) inclut une seconde partie terminale (3d) à laquelle la partie de connexion (4) est connectée de manière détachable.

6. Dispositif de mesure d'informations biologiques (1) selon l'une quelconque des revendications 1 à 5, dans lequel
le boîtier (2a) est reliée de manière détachable à la première partie d'ajustement (2b).

7. Dispositif de mesure d'informations biologiques (1) selon l'une quelconque des revendications 1 à 6, dans lequel
la partie de connexion (4) relie le corps (2) et l'unité de sonde (3) d'une manière telle qu'une relation de position entre le corps (2) et l'unité de sonde (3) peut être changée.

8. Dispositif de mesure d'informations biologiques (1) selon la revendication 7, dans lequel la partie de connexion (4) est configurée pour être courbée pour changer la relation de position entre le corps (2) et l'unité de sonde (3).

9. Dispositif de mesure d'informations biologiques (1) selon la revendication 8, dans lequel
lorsque la première partie d'ajustement (2b) et la seconde partie d'ajustement (3c) sont portées sur un ou plusieurs doigts du corps du sujet, la partie de connexion (4) est configurée pour être disposée le long d'une partie ventrale du doigt (F1) qui est positionnée à l'intérieur lorsque le doigt (F1) est plié.

10. Dispositif de mesure d'informations biologiques (1) selon l'une quelconque des revendications 1 à 9, dans
lequel le corps (2) inclut en outre une unité de communication (24) configurée pour transmettre des données par voie sans fil.

11. Oxymètre de pouls configuré en tant que dispositif de mesure d'informations biologiques (1) selon l'une quelconque des revendications 1 à 10 et
configuré pour acquérir des informations sur au moins la saturation en oxygène dans le sang du corps du sujet en tant qu'informations biologiques.
